# EUROPÄISCHE PATENTSCHRIFT

(11) **EP 0 677 097 B1**
(45) Veröffentlichungstag und Bekanntmachung des Hinweises auf die Patenterteilung: **15.01.1997**
(21) Anmeldenummer: 94903826.9
(22) Anmeldetag: 17.12.1993
(51) Int. Cl.: C12C 11/07, C12C 12/04

(54) **VERFAHREN ZUR HERSTELLUNG VON BIER**
BEER PRODUCTION PROCESS
PROCEDE DE PRODUCTION DE BIERE

(30) Priorität: 31.12.1992 DE 4244595; 31.12.1992 DE 4244596; 31.12.1992 DE 4244597
(43) Veröffentlichungstag der Anmeldung: 18.10.1995
(73) Patentinhaber: METALLGESELLSCHAFT AKTIENGESELLSCHAFT, 60623 Frankfurt am Main (DE)
(72) Erfinder: DZIONDZIAK, Klaus, D-25421 Pinneberg (DE); BÖNSCH, Rudolf, D-55299 Nackenheim (DE); BODMER, Roland, D-61130 Nidderau (DE); EICHELSBACHER, Michael, D-55130 Mainz (DE); MITSCHKE, Peter, D-63477 Maintal (DE); SANDER, Ulrich, D-61381 Friedrichsdorf (DE); SCHLICHTING, Eberhard, D-61273 Wehrheim (DE)
(86) Internationale Anmeldenummer: EP9303601
(87) Internationale Veröffentlichungsnummer: WO9416054

(56) Entgegenhaltungen:
- EP-A- 0 245 845
- DE-A- 2 429 574
- GB-A- 943 091
- GB-A- 1 211 576
- GB-A- 2 010 326
- US-A- 5 242 694

## Beschreibung

Die Erfindung bezieht sich auf ein Verfahren zur Herstellung von Bier, bei dem stärkehaltige Rohstoffe zerkleinert und ggf. zu Malz verarbeitet, aus den zerkleinerten und ggf. gemälzten Rohstoffen eine Würze gewonnen und die Würze der alkoholischen Gärung unterworfen wird. Bier ist ein Sammelbegriff für alle aus stärkehaltigen Rohstoffen durch alkoholische Gärung gewonnenen Getränke, wobei dem Bier nach der Gärung der Alkohol ganz oder teilweise entzogen werden kann. Alkoholhaltiges Bier (Schank- oder Vollbier) enthält mehr als 2,5 Vol.% Alkohol; alkoholarmes Bier enthält weniger als 2,5 Vol.%, vorzugsweise weniger als 1 Vol.% Alkohol; alkoholfreies Bier enthält weniger als 0,05 Vol.% Alkohol.

Stärkehaltige Rohstoffe können nicht direkt vergoren werden, sondern sie sind vorher zu "verzuckern". Dabei sind hydrolytisch wirkende Enzyme (Amylasen, Proteasen, Glucanasen) aktiv. Die Stärke wird bei der Verzuckerung in Dextrine und gärungsfähige Zucker überführt. In der Regel wird zur Herstellung von Bier Malz verwendet, das üblicherweise aus geeigneten Gerstensorten in der Weise gewonnen wird, daß die Gerste zunächst mehrere Tage bei 15 bis 18°C zum Keimen gebracht wird und daß anschließend eine Trocknung der gekeimten Gerste bei Temperaturen bis zu 100°C erfolgt. Das von Staub und Keimen befreite Malz, in dem die Stärke bereits teilweise in Form von Dextrinen und Maltose vorliegt und das außerdem arteigene hydrolytisch wirkende Enzyme enthält, die während der Würzebereitung die Verzuckerung der Stärke zum Abschluß bringen, wird zerkleinert. Das zerkleinerte Malz wird mit Wasser eingemaischt und bei 35 bis 75°C behandelt, wobei die Verzuckerung zum Abschluß kommt und die Dextrine sowie die vergärbaren Zucker in Lösung gehen. Nach Abtrennung der unlöslichen Bestandteile (Treber) wird die Würze gekocht. Vor und/oder während der Kochung erfolgt die Aromatisierung der Würze mit Hopfen oder Hopfenextrakt. Nach Abtrennung von Trubstoffen und Kühlung der gehopften Würze wird der für die Gärung erforderliche Sauerstoff zugesetzt, und es schließt sich die Gärung an, die bei 6 bis 20°C während mehrerer Tage durchgeführt wird. Die Nachgärung erfolgt bei -2 bis 3°C. Anschließend wird das Bier filtriert und ggf. mit CO₂ versetzt. Werden anstelle von Malz ungemälzte Cerealien oder andere stärkehaltige Rohstoffe verwendet, so erfolgt die Verzuckerung in speziellen Fällen in der Weise, daß die stärkehaltigen aufgemahlenen Rohstoffe mit Wasser vermischt und anschließend durch nichtarteigene, hydrolytisch wirkende Enzyme aufgeschlossen - also in Dextrine und gärungsfähige Zucker - überführt werden.

Alle Verfahrensstufen der Bierherstellung werden normalerweise im Batchbetrieb durchgeführt. Um die Wirtschaftlichkeit der Bierherstellung zu verbessern, wurde bereits ein teilkontinuierlicher bzw. kontinuierlicher Braubetrieb vorgeschlagen. Die kontinuierliche Biergewinnung unter Verwendung von immobilisierten Hefezellen, die seit einigen Jahren intensiv untersucht wird, ist aber immer wieder aus unterschiedlichen Gründen als nicht restlos befriedigend kritisiert worden.

Die Veröffentlichung von P. Kollnberger, Brauindustrie 6/91, Seiten 514 bis 520, gibt einen allgemeinen Überblick über kontinuierliche Brauverfahren und kommt zum Ergebnis, daß Gesamtanlagen der kontinuierlichen Bierherstellung bisher nicht bekannt geworden sind, da die Bierherstellung von zu vielen Faktoren abhängig ist, um einen kontinuierlichen Gesamtprozeß zu rechtfertigen.

Aus der Veröffentlichung von L. Ehnstrom, Food Engineering int'1., Dezember 1976, Seiten 22 bis 27, ist ein kontinuierliches Verfahren zur Würzegewinnung bekannt, bei dem die Rohstoffe bis zu einer Teilchengröße von 100 bis 500 µm trockengemahlen werden, bei dem das Maischen in einem Rohrreaktor erfolgt, bei dem die Läuterung durch Gegenstromextraktion in mehreren Separatoren durchgeführt wird und bei dem die Kochung durch direktes Einblasen von Dampf unter Druck bei 140 bis 150°C sowie anschließendes Entspannen in ein Vakuum bis zu einer Temperatur von 65 bis 95°C ausgeführt wird.

Die Veröffentlichung von S. Julin und H. Berger, Brauwelt 15, 1979, Seiten 492 bis 494, schlägt eine Hochtemperatur-Würzekochung vor, bei der die gehopfte Würze in drei Spiralwärmeaustauschern stufenweise auf eine Temperatur von ca. 140°C gebracht und nach Durchlaufen einer Reaktionsstrecke, die eine Heißhaltezeit von 5 Minuten gewährleistet, in zwei Stufen auf Umgebungsdruck entspannt wird, wobei sich eine Temperatur von ca. 100°C in der gekochten Würze einstellt.

Die Veröffentlichungen von Julin und Berger sowie von Ehnstrom offenbaren also Teilprozesse der kontinuierlichen Bierherstellung; sie machen aber keine Vorschläge zur Einkopplung der kontinuierlichen Würzeherstellung in ein kontinuierliches Gär- und Reifeverfahren.

Die DE-OS 18 04 343 betrifft eine Vorrichtung zur Trennung der Würze von den Trebern, die im wesentlichen aus einer Maischpfanne und zwei nachgeordneten Extrakteuren besteht. Die Extrakteure sind jeweils in eine vordere und eine rückwärtige Kammer unterteilt, wobei die Maische kontinuierlich von der Maischpfanne aus durch die Extrakteure zum Treberaustrag befördert wird. Der deutschen Offenlegungsschrift ist nicht zu entnehmen, ob und ggf. wie die dort vorgeschlagene Abtrennung der Würze von den Trebern in einen kontinuierlichen Brauprozeß eingekoppelt werden kann.

In der AT-PS 289 685 wird ein Verfahren zum kontinuierlichen Vergären von Bierwürze vorgeschlagen, das bei ca. 10°C abläuft und bei dem die in Gärung befindliche Würze innerhalb eines Behälters unter Druck vergoren wird, wobei Strömungsgeschwindigkeit und Strömungsrichtung praktisch in jedem Bereich des Gärbehälters konstant sind. Die im Behälter befindliche Fermentationsflüssigkeit ist als eine aus Würze und Kohlensäure bestehende Emulsion konstanter Zusammensetzung ausgebildet, deren spezifisches Gewicht einen konstanten Wert zwischen 0,15 und 0,40 g/cm³ hat, was auf einen außerordentlich hohen CO₂-Gehalt hindeutet, der sowohl verfahrenstechnische Nachteile als auch Qualitätseinbußen des fertigen Bieres zur Folge hat. Bei diesem Verfahren ist auch vorgesehen, daß die aufsteigende Bewegung innerhalb des Behälters durch Einblasen von CO₂ erzeugt wird, das dem oberen Teil des Reaktors entnommen wurde.

Die DE-PS 41 42 646 offenbart ein Verfahren zur Herstellung eines alkoholarmen Bieres mit einem Alkoholgehalt < 0,5 Gew.%, bei dem eine erste Vollbierwürze weitestgehend durchgegoren und das dabei erhaltene Produkt auf einen Alkoholgehalt < 0,5 % entalkoholisiert wird, bei dem eine zweite Vollbierwürze mit Brauwasser bis auf einen Stammwürzegehalt von Einfachbier verschnitten und das dabei erhaltene Produkt bis auf einen Alkoholgehalt von < 0,5 % durchgegoren wird und bei dem das entalkoholisierte Vollbier sowie das Einfachbier zu einem alkoholarmen Schankbier verschnitten werden. Die DE-PS 41 42 646 offenbart kein kontinuierliches Verfahren zur Bierherstellung.

Schließlich ist es bekannt, aus dem alkoholhaltigen Bier den Alkohol abzutrennen, was durch Entspannungsverdampfung, Strippen mit Gasen oder Permeation an einer Membran erfolgen kann. So wird beispielsweise in der EP-OS 0 245 845 ein Verfahren zur Herstellung von alkoholfreien Bieren vorgeschlagen, bei dem zur Desorption des Alkohols ein Gas durch ein alkoholhaltiges Bier geleitet wird und bei dem der auftretende Geschmacksverlust des Bieres durch Stoffe ausgeglichen wird, die den Geschmack des Bieres verbessern. Als Gas kann bei dem bekannten Verfahren auch Luft verwendet werden. Der Erfindung liegt die Aufgabe zugrunde, ein kontinuierlich arbeitendes Verfahren zur Herstellung von Bier zu schaffen, das über einen langen Zeitraum die Produktion eines Bieres von hoher und gleichbleibender Qualität gestattet und das auch die kontinuierliche Herstellung eines alkoholarmen oder alkoholfreien Bieres hoher Qualität ermöglicht, wobei die einzelnen Schritte des Brauverfahrens sowie der teilweisen oder vollständigen Entalkoholisierung des Bieres so miteinander verknüpft werden, daß niedrige Produktionskosten resultieren.

Die der Erfindung zugrundeliegende Aufgabe wird dadurch gelöst, daß
a) zerkleinerte, stärkehaltige und ggf. gemälzte Rohstoffe mit Wasser eingemaischt und die Maische mindestens einem Reaktor kontinuierlich zugeführt wird, wobei die Temperatur der Maische vor Eintritt in mindestens einen der Reaktoren durch indirekten Wärmeaustausch stufenweise auf eine Endtemperatur von 75 bis 85°C angehoben wird, wobei die Verweilzeit in den Reaktoren 30 bis 90 Minuten beträgt und wobei die Maische in den einzelnen Reaktoren auf einem definierten Temperaturniveau gehalten wird,
b) der Treber aus der Maische in einem Dekanter kontinuierlich abgetrennt und anschließend mit dem Brauwasser in einem zweistufigen Dekanter ausgelaugt wird,
c) die feststofffreie, heiße Würze mit Hopfen oder Hopfenextrakt gemischt sowie kontinuierlich einem Durchflußreaktor zugeführt und auf eine Temperatur von 105 bis 140°C erhitzt wird sowie während der Durchgangszeit durch den Reaktor von 2 bis 60 Minuten auf dieser Temperatur und auf einem Druck von 1,2 bis 3,6 bar gehalten wird,
d) die unter Druck stehende Würze einer Entspannungsverdampfung unterworfen, in einem Separator kontinuierlich von den Trubstoffen befreit und anschließend in einem Wärmeaustauscher auf die Vergärungstemperatur abgekühlt wird,
e) die abgekühlte Würze mit einem Sauerstoffgehalt von 0,5 bis 3,0 mg O₂/l mindestens einem als Schlaufenreaktor gestalteten Fermenter kontinuierlich zugeführt wird, der bei einer Temperatur von 6 bis 25°C sowie einem Druck von 1,5 bis 2 bar arbeitet, in dem die Würze eine mittlere Verweilzeit von 1 bis 40 Stunden hat sowie ständig im Kreislauf geführt wird und in dem sich ein Biokatalysator befindet, der eine biologisch aktive Hefe enthält,
f) während der Gärung kontinuierlich flüssiges Medium aus dem Fermenter abgezogen, zur Entfernung der darin befindlichen freien Hefezellen zentrifugiert, das enthefte flüssige Medium während 0,5 bis 30 Minuten auf 50 bis 90°C erhitzt und in zwei Teilströme heißen Bieres aufgeteilt wird,
g) ein Teilstrom des in der Verfahrensstufe f) anfallenden heißen Bieres abgekühlt und in den Fermenter zurückgeführt wird,
h) der zweite Teilstrom entweder teilweise oder ganz entalkoholisiert und als alkoholarmes oder alkoholfreies Bier abgegeben wird oder abgekühlt, filtriert, mit CO₂ versetzt und als alkoholhaltiges Bier abgegeben wird.

Das erfindungsgemäße Verfahren eignet sich in vorteilhafter Weise sowohl zur Verarbeitung von Malz, wodurch die Einhaltung des deutschen Reinheitsgebotes erreicht wird, als auch zur Verarbeitung anderer stärkehaltiger Rohstoffe, wie z.B. Mais oder Sorghum. Nichtvermälzte, stärkehaltige Rohstoffe, wie z.B. Mais oder Sorghum, können, thermisch und/oder enzymatisch, vorbehandelt werden. Der kombinierte Einsatz von Durchflußreaktoren, Dekantern, Biokatalysatoren und nach dem Prinzip des Schlaufenreaktors arbeitenden Wirbelbett-Fermentern gestattet eine kontinuierliche Prozeßführung. Dabei können Fremdinfektionen bei Betriebszeiten von mehr als 8000 Stunden wirksam beherrscht werden. Insbesondere wird durch die Abtrennung der freien Hefezellen eine optimale Nährstoffzufuhr in den Biokatalysator sowie eine Erhaltung der Langzeitaktivität und der Struktur des Biokatalysators erreicht. Ferner ermöglicht das erfindungsgemäße Verfahren eine optimale Rohstoff- und Energieausnutzung bei geringen Investitionskosten. Die gute geschmackliche Qualität des nach dem erfindungsgemäßen Verfahren hergestellten Biers wird insbesondere darauf zurückgeführt, daß durch die Entspannung der entheften, wärmebehandelten Würze gemäß Verfahrensstufe d) eine vorteilhafte Abtrennung des durch thermische Umwandlung von δ-Acetolactat gebildeten Diacetyls erreicht wird. Trotz der verhältnismäßig kurzen Vergärungszeiten wird ein weitgehender oder nahezu vollständiger Umsatz der vergärbaren Zucker zu Alkohol erreicht, so daß sich das erfindungsgemäße Verfahren zur Herstellung sowohl von Schankbier (ca. 2,5 bis 3,0 Vol.% Alkohol) als auch zur Herstellung von Vollbier (ca. 4,5 bis 5,0 Vol.% Alkohol) eignet. Die teilweise oder vollständige Entalkoholisierung des Bieres ist auf kontinuierlichem Weg ohne Qualitätsverlust möglich und kann vorteilhaft in den kontinuierlichen Brauprozeß eingekoppelt werden.

Es hat sich nach der Erfindung als besonders vorteilhaft erwiesen, wenn das Einmaischen der zerkleinerten Rohstoffe gemäß Verfahrensstufe a) in einer Kolloidmühle erfolgt. In der Kolloidmühle wird nicht nur eine gute Durchmischung, sondern auch eine nochmalige Zerkleinerung der eingemaischten Rohstoffe erreicht.

Das erfindungsgemäße Verfahren kann besonders vorteilhaft durchgeführt werden, wenn die Verfahrensstufe c) bei einer Temperatur von 110 bis 125°C sowie einem Druck von 1,4 bis 2,3 bar durchgeführt wird.

Ferner ist in weiterer Ausgestaltung der Erfindung vorgesehen, daß die Entspannungsverdampfung gemäß Verfahrensstufe d) in zwei Stufen erfolgt, wobei in der ersten Stufe auf 1 bar und in der zweiten Stufe auf 0,3 bis 0,7 bar entspannt wird. Die zweistufige Entspannungsverdampfung ermöglicht eine optimale Wärmerückgewinnung. Nach der Erfindung hat es sich als besonders vorteilhaft erwiesen, wenn während der Entspannungsverdampfung CO₂ zugeführt wird, denn durch diese Maßnahme werden noch vor der Vergärung unerwünschte Geruchs- und Geschmacksstoffe aus der Würze abgetrieben.

In einigen Fällen hat es sich als zweckmäßig erwiesen, wenn die Würze nach Abtrennung der Trubstoffe gemäß Verfahrensstufe d) in einen Lagerbehälter geführt wird und wenn die dem Lagerbehälter entnommene Würze vor ihrem Eintritt in den Wärmeaustauscher der Stufe d) kurzfristig auf 60 bis 100°C erhitzt wird. Hierdurch kann im Bedarfsfall, z.B. bei einer Betriebsstörung oder während Reinigungsarbeiten, eine längere Lagerung der vergärungsfähigen Würze erfolgen, ohne daß eine Infektion der Fermentationsstufe durch die in der gelagerten Würze möglicherweise enthaltenen Mikroorganismen zu befürchten wäre.

Nach der Erfindung wird ein besonders störungsfreier und vollständiger Gärungsverlauf erreicht, wenn die Würze nacheinander durch drei Fermenter geführt wird, wobei die Verweilzeit in den Fermentern insgesamt 10 bis 40 Stunden beträgt. Alternativ ist nach der Erfindung vorgesehen, daß die Würze lediglich durch einen Fermenter geführt wird, in dem die Verweilzeit 1 bis 8 Stunden beträgt. Diese Prozeßführung hat sich zur Herstellung eines qualitativ hochwertigen alkoholarmen oder alkoholfreien Bieres bewährt, denn bereits während der relativ kurzen Vergärungszeit wird eine weitgehende Vergärung der in der Würze enthaltenen Zucker erreicht.

Nach der Erfindung ist es besonders vorteilhaft, wenn der Biokatalysator einen TiO₂,-Gehalt von 5 bis 30 Gew.% aufweist sowie eine biologisch aktive Hefe und eine gelartige Matrix enthält, wobei die TiO₂-Teilchen einen Durchmesser von 0,1 bis 1 µm haben und der Katalysator kugelförmig ist. Der Biokatalysator hat den Vorteil, daß er gleichmäßig im Wirbelbett des Fermenters verteilt werden kann, gute mechanische Festigkeitseigenschaften besitzt und lediglich Substanzen enthält, die natürlichen Ursprungs sind bzw. die sich in chemischen bzw. biologischen Reaktionssystemen inert verhalten.

Nach der Erfindung ist es zweckmäßig, wenn das enthefte flüssige Medium gemäß Verfahrensstufe f) während 15 bis 20 Minuten auf 60 bis 65°C erhitzt wird; durch diese Behandlung tritt keine negative Geschmacksveränderung des Bieres ein.

Zur Herstellung eines alkoholarmen Bieres ist nach der Erfindung vorgesehen, daß der in der Verfahrensstufe f) anfallende zweite Teilstrom des heißen Bieres nochmal in zwei Teile geteilt wird, wobei aus dem ersten Teil des heißen Bieres der Alkohol kontinuierlich durch Strippen mit Luft und/oder Wasserdampf oder durch Entspannungsverdampfung entfernt und das entalkoholisierte Bier mit dem zweiten Teil verschnitten wird, wobei das Mengenverhältnis des ersten und zweiten Teils so gewählt wird, daß in der Mischung ein Alkoholgehalt < 2,5 Vol.%, vorzugsweise < 1 Vol.%, resultiert und daß das heiße alkoholarme Bier abgekühlt, filtriert und mit CO₂ versetzt wird.

Zur Herstellung eines alkoholfreien Bieres ist nach der Erfindung vorgesehen, daß aus dem in der Verfahrensstufe f) anfallenden zweiten Teilstrom des heißen Bieres der Alkohol kontinuierlich durch Strippen mit Luft und/oder Wasserdampf oder durch Entspannungsverdampfung entfernt wird und daß das alkoholfreie Bier abgekühlt, filtriert und mit CO₂ versetzt wird.

Die gute geschmackliche Qualität des nach dem erfindungsgemäßen Verfahren hergestellten alkoholarmen und alkoholfreien Bieres wird insbesondere darauf zurückgeführt, daß durch das Strippen oder durch die Entspannungsverdampfung nicht nur der Alkohol abgetrennt, sondern in vorteilhafter Weise auch die Abtrennung des durch thermische Umwandlung von δ-Acetolactat gebildeten Diacetyls erreicht wird, während die biertypischen Geschmacksstoffe erhalten bleiben. Eine der geschmacklichen Verbesserung des alkoholarmen oder alkoholfreien Bieres dienende zeitaufwendige Reifung ist also nicht erforderlich. Da durch die erfindungsgemäße Entalkoholisierung ein Produkt mit sehr niedrigem Alkoholgehalt erhalten wird, kann das entalkoholisierte Bier mit einer nennenswerten Menge alkoholhaltigen Bieres verschnitten werden, um beispielsweise ein alkoholarmes Bier mit einem Alkoholgehalt < 0,5 Vol.% herzustellen, das gemäß den deutschen lebensmittelrechtlichen Bestimmungen noch als alkoholfrei gilt. Durch das nach dem erfindungsgemäßen Verfahren vorgesehene Verschneiden wird erreicht, daß während des Brauprozesses gebildete erwünschte Geruchs- und Geschmacksstoffe in den alkoholfreien Teilstrom eingetragen werden und dessen Geschmack verbessern.

Die Entalkoholisierung des Bieres erfolgt erfindungsgemäß in vorteilhafter Weise dadurch, daß der Alkohol aus dem 50 bis 60°C heißen Bier durch Strippen mit Luft in einer Kolonne entfernt wird, wobei die Luft mit Wasserdampf angereichert ist und eine Temperatur von 60 bis 100°C hat, und daß das alkoholfreie Bier anschließend auf 1 bar entspannt wird. Diese Verfahrensführung gestattet eine weitgehend quantitative Abtrennung des Alkohols, ohne daß dabei eine negative Geschmacksveränderung des Biers eintritt. Zur Entalkoholisierung des Biers werden vorzugsweise 3 bis 6 Nm³ Luft pro Liter Bier benötigt. Der Wasserdampfgehalt der Luft liegt vorzugsweise bei 85 bis 95 % der Sättigungskonzentration, bezogen auf die Temperatur der Luft am Stripperaustritt. Ein merklicher Verlust an erwünschten Geruchs- und Geschmacksstoffen tritt während des Strippens nicht ein.

Nach der Erfindung ist es in einigen Fällen zweckmäßig, wenn das alkoholarme oder alkoholfreie abgekühlte Bier einem als Schlaufenreaktor gestalteten Fermenter zugeführt wird, der bei einer Temperatur von 0 bis 6°C sowie einem Druck von 1,2 bis 1,5 bar arbeitet, in dem das alkoholfreie Bier eine Aufenthaltszeit von 0,2 bis 2 Stunden hat und in dem sich ein Biokatalysator befindet, der mit dem in Verfahrensstufe e) verwendeten Biokatalysator identisch ist. Durch diese Nachgärung des alkoholfreien Bieres bei tiefer Temperatur tritt keine nennenswerte Erhöhung des Alkoholgehalts ein; allerdings kann durch die Nachgärung die geschmackliche Qualität des alkoholarmen und des alkoholfreien Biers noch weiter verbessert werden.

Der Gegenstand der Erfindung wird nachfolgend anhand der in der Zeichnung dargestellten Verfahrensfließbilder näher erläutert. Es zeigen:
- Fig. 1: Verfahren zur Herstellung eines Vollbiers mit einem Alkoholgehalt von ca. 4,5 Vol.%,
- Fig. 2: Verfahren zur Herstellung eines alkoholarmen Biers mit einem Alkoholgehalt von ca. 0,5 Vol.%,
- Fig. 3: Verfahren zur Herstellung eines alkoholfreien Biers mit einem Alkoholgehalt von ca. 0,05 Vol.%.

Bei allen drei Verfahrensvarianten wird Braugerste befeuchtet und bei einer Temperatur von 15 bis 18°C innerhalb von 8 Tagen gekeimt, wobei sich das Grünmalz bildet. Das Grünmalz wird an der Luft getrocknet und anschließend auf Horden bei 50 bis 85°C gedarrt. Das dabei gebildete Darrmalz wird von Keimen und Staub befreit sowie anschließend geschrotet. Beim Keimen und Darren bilden sich arteigene, hydrolytisch wirkende Enzyme, welche die in der Braugerste enthaltene Stärke teilweise verzuckern. Die vorstehend beschriebenen Arbeitsgänge sind in den Verfahrensfließbildern nicht dargestellt.

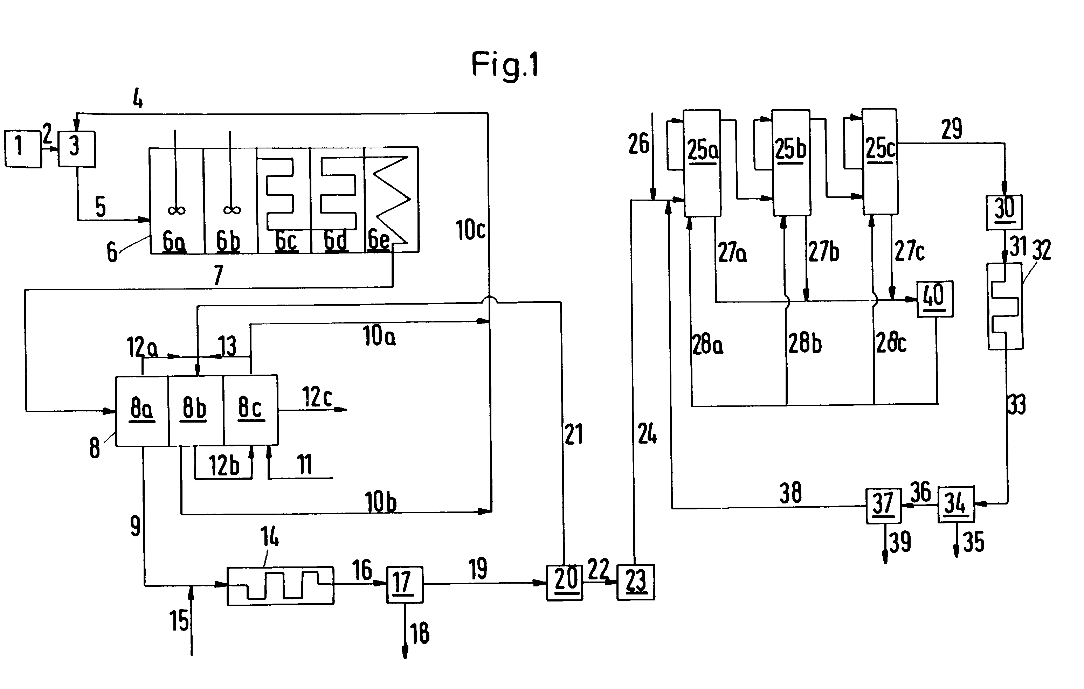

Das Vollbier wird gemäß dem in Fig. 1 dargestellten Verfahren wie folgt hergestellt:

Aus dem Vorratstank (1) wird das geschrotete Malz über die Leitung (2) kontinuierlich in die Kolloidmühle (3) gefördert, der außerdem Brauwasser über die Leitung (4) kontinuierlich zugeführt wird. In der Kolloidmühle (3) wird das geschrotete Malz nochmal zerkleinert und gleichzeitig intensiv mit dem Brauwasser gemischt. Das Brauwasser wird in der Leitung (4) geführt und hat eine Temperatur von ca. 35°C, so daß die Maische die Kolloidmühle (3) mit einer Temperatur von 35°C verläßt.

Die Maische tritt über die Leitung (5) kontinuierlich in das Reaktorsystem (6) ein, das aus den beiden Rührreaktoren (6a) und (6b), den beiden Durchflußreaktoren (6c) und (6d) sowie dem Wärmeaustauscher (6e) besteht. Am Eintritt des Reaktorsystems (6) hat die Maische eine Temperatur von 35°C; sie wird im Rührreaktor (6a) bei dieser Temperatur etwa 20 Minuten gehalten. Die Maische fließt dann in den Rührreaktor (6b) und hat hier bei einer Temperatur von 50°C eine Verweilzeit von ca. 20 Minuten (Proteaserast). Im Durchflußreaktor (6c) wird die Maische während 16 Minuten bei einer Temperatur von 63°C gehalten (β-Amylaserast); es schließt sich im Durchflußreaktor (6d) die δ-Amylaserast an, die während 8 Minuten bei 73°C abläuft. In einem nachgeschalteten Wärmeaustauscher (6e) wird die Maische auf 76 bis 78°C erhitzt, wobei die Enzyme inaktiviert werden. Die einzelnen Apparate des Reaktorsystems (6) erlauben die Einhaltung einzelner Rastzeiten bei definierten Temperaturen. Die den einzelnen Reaktoren vorgeschalteten Wärmeaustauscher, in denen die definierten Temperaturen für die Rastzeiten eingestellt werden, sind in der Zeichnung nicht dargestellt.

Die Maische wird dem Reaktorsystem (6) kontinuierlich entnommen und über die Leitung (7) in einen dreistufigen Dekanter (8) gefördert, in dem eine Fest-Flüssig-Trennung nach dem Prinzip des Zentrifugierens erfolgt. In der ersten Dekanterstufe (8a) wird der Treber abgetrennt und über die Leitung (12a) in die zweite Dekanterstufe (8b) gefördert. In der zweiten Dekanterstufe (8b) erfolgt die Auslaugung des Trebers, wobei der zweiten Dekanterstufe (8b) ein Teilstrom des Ablaufs der dritten Dekanterstufe (8c) über die Leitung (13) zugeführt wird. Der ausgelaugte Treber wird über die Leitung (12b) aus der zweiten Dekanterstufe (8b) in die dritte Dekanterstufe (8c) geführt, wo eine nochmalige Auslaugung stattfindet. Die in der dritten Dekanterstufe (8c) ablaufende Auslaugung wird mit Wasser durchgeführt, das der dritten Dekanterstufe (8c) über die Leitung (11) zugeführt wird. Der Ablauf der zweiten Dekanterstufe (8b) sowie der nicht zur Auslaugung verwendete Teilstrom des Ablaufs der dritten Dekanterstufe (8c) werden über die Leitungen (10a), (10b) und (10c) in die Leitung (4) gefördert; beide Ströme werden also als Brauwasser genutzt. Aus der dritten Dekanterstufe (8c) wird der zweifach ausgelaugte Treber über die Leitung (12c) abgeführt.

Die feststofffreie, heiße Würze wird aus der Leitung (9) kontinuierlich in den als beheizbaren Röhrenreaktor gestalteten Durchflußreaktor (14) gefördert, wo eine Kochung bei 115°C während einer Zeit von ca. 30 Minuten erfolgt. Während der Kochung wird ein Druck von ca. 1,7 bar eingestellt. Vor dem Eintritt der Würze in den Durchflußreaktor (14) wird sie mit Hopfenextrakt versetzt, der über die Leitung (15) zudosiert wird. Bei der Kochung der gehopften Würze erfolgt eine Isomerisierung von Inhaltsstoffen des Hopfenextrakts sowie eine Koagulation von Eiweißkörpern.

Aus dem Durchflußreaktor (14) wird die unter Druck stehende Würze über die Leitung (16) kontinuierlich in den Entspannungsverdampfer (17) gefördert, wo unter Abkühlung eine Entspannung auf 1 bar (Atmosphärendruck) erfolgt. Das Brüdenkondensat gelangt über die Leitung (18) in den Abwasserkanal, während die Würze über die Leitung (19) kontinuierlich in den Separator (20) gefördert wird, wo die Abtrennung der Trubstoffe erfolgt, die über die Leitung (21) ausgetragen werden. Die Trubstoffe werden zur Nutzung löslicher Inhaltsstoffe der zweiten Dekanterstufe (8b) zugeführt und dort ausgelaugt. Die feststofffreie Würze gelangt dann über die Leitung (22) in den Wärmeaustauscher (23), wo eine Abkühlung auf die Vergärungstemperatur von 16°C erfolgt. Es ist möglich, die aus dem Wärmeaustauscher (23) abfließende gekühlte Würze in einen Lagerbehälter zu fördern. Der Lagerbehälter hat die Aufgabe, während der Reinigung der Kochapparate dem Fermentersystem kontinuierlich Würze zuzuführen. Dem Lagerbehälter kann auch ein Kurzzeiterhitzer nachgeschaltet werden, in dem die Würze erhitzt und damit entkeimt wird. In der Zeichnung sind Lagerbehälter und Kurzzeiterhitzer nicht dargestellt.

Aus dem Wärmeaustauscher (23) fließt die abgekühlte Würze über die Leitung (24) kontinuierlich in das aus drei Fermentern (25a), (25b) und (25c) bestehende Fermentersystem. Jeder Fermenter arbeitet nach dem Prinzip des Schlaufenreaktors, d.h., daß die Würze innerhalb des Fermenters im Kreislauf geführt wird, wobei das Verhältnis von Zulauf und Umlauf zwischen 1 : 30 und 1 : 80 liegt. Die Reaktionszone jedes Fermenters enthält einen Biokatalysator, der durch die umlaufende Würze im Wirbelzustand gehalten wird. Jeder Fermenter wird bei einem Druck von etwa 1,8 bar gefahren. Die in den ersten Fermenter eintretende gekühlte Würze wird in der Leitung (24) mit Luft gemischt, wobei ein Sauerstoffgehalt von 1,5 mg O₂/l Würze einzustellen ist. Die Luft wird über die Leitung (26) zugeführt. Das flüssige Medium hat in jedem Fermenter eine Aufenthaltszeit von ca. 10 Stunden, so daß eine Vergärungszeit von insgesamt 30 Stunden erreicht wird, in der der weitaus größte Teil der vergärbaren Zucker in Alkohol und CO₂ umgewandelt wird. Auf diese Weise wird ein Bier erhalten, dessen Alkoholgehalt durch den Extraktgehalt und den Vergärungsgrad definiert ist. Als Biokatalysator hat sich in vorteilhafter Weise der in der DE-PS 3 704 478 beschriebene Katalysator wegen seiner guten mechanischen Festigkeit und seines optimalen Wirbelbettverhaltens bewährt.

In einigen Fällen ist es vorteilhaft, daß jedem der drei Fermenter ein Teilstrom der in der Leitung (24) geführten, abgekühlten, sauerstoffhaltigen Würze zugeführt wird, wobei das Mengenverhältnis der drei Teilströme bei 75 : 15 : 10 liegt, so daß auch dem dritten Fermenter noch 10 % der Würze zugeführt werden. Diese Prozeßführung ermöglicht einen störungsfreien Betrieb der Fermenter; sie ist in der Zeichnung aber nicht dargestellt.

Während der Vergärung kommt es zur Bildung von Hefezellen, die nicht im Biokatalysator immobilisiert sind, sondern mit dem flüssigen Medium eine Suspension bilden. Es ist vorteilhaft, die freien Hefezellen aus dem Inhalt jedes Fermenters durch Abzentrifugieren eines Teilstroms abzutrennen. Dazu wird den einzelnen Fermentern (25a), (25b) und (25c) ein Teilstrom über die Leitungen (27a), (27b) und (27c) entnommen, über die Zentrifuge (40) geführt, und der jeweilige Teilstrom gelangt über die Leitungen (28a), (28b) und (28c) in den jeweiligen Fermenter zurück. Der Ablauf des letzten Fermenters (25c) wird kontinuierlich über die Leitung (29) in die Zentrifuge (30) gefördert, wo eine Abtrennung eventuell vorhandener freier Hefezellen erfolgt. Die Hefezellen werden der Zentrifuge (30) entnommen, was in der Zeichnung nicht dargestellt ist. Durch das Abzentrifugieren der freien Hefezellen in der Zentrifuge (30) wird auch eine signifikante Verbesserung der abschließenden Filtration des Biers erreicht.

Das vergorene flüssige Medium gelangt dann über die Leitung (31) in den Durchflußreaktor (32), wo es während 15 Minuten auf eine Temperatur von 65°C erhitzt wird. Der Durchflußreaktor (32) ist über die Leitung (33) mit dem Entspannungsverdampfer (34) verbunden, in dem eine Entspannung auf 1 bar (Atmosphärendruck) erfolgt. Das Brüdenkondensat des Entspannungsverdampfers (34) wird über die Leitung (35) in den Abwasserkanal abgegeben, während das Bier über die Leitung (36) in den Wärmeaustauscher (37) gelangt, wo eine Abkühlung auf ca. 15°C erfolgt. Ein Teilstrom des den Wärmeaustauscher (37) verlassenden Bieres wird dem Fermenter (25a) über die Leitung (38) zugeführt, während der zweite Teilstrom den Wärmeaustauscher (37) als fertiges Produkt über die Leitung (39) verläßt. Im Bedarfsfall ist es möglich, den in der Leitung (39) geführten Ablauf des Wärmeaustauschers (37), ggf. unter Zusatz eines Filterhilfsmittels, zu filtrieren. Dieser Verfahrensschritt ist in der Zeichnung nicht dargestellt.

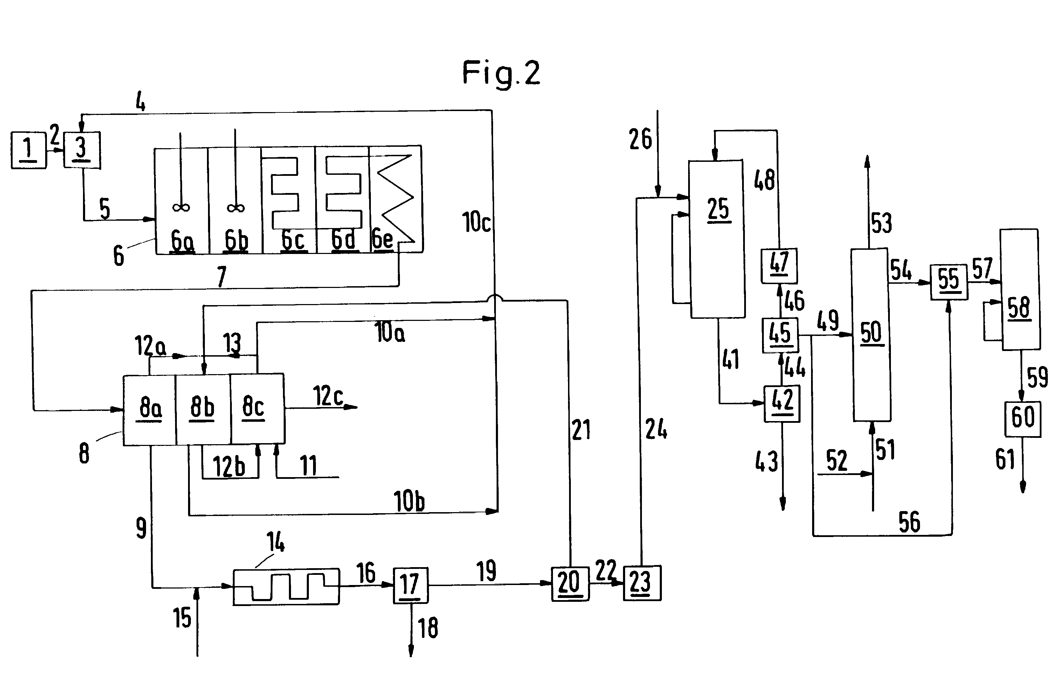

Das alkoholarme Bier wird gemäß dem in Fig. 2 dargestellten Verfahren wie folgt hergestellt:

Die feststofffreie Würze wird gemäß dem in Fig. 1 dargestellten und vorstehend beschriebenen Teilprozeß hergestellt und im Wärmeaustauscher (23) auf die Vergärungstemperatur von 16°C abgekühlt. Aus dem Wärmeaustauscher (23) fließt die abgekühlte Würze über die Leitung (24) kontinuierlich in den Fermenter (25), der nach dem Prinzip des Schlaufenreaktors arbeitet, d.h., daß die Würze innerhalb des Fermenters im Kreislauf geführt wird, wobei das Verhältnis von Zulauf und Umlauf zwischen 1 : 30 und 1 : 80 liegt. Die Reaktionszone des Fermenters (25) enthält einen Biokatalysator, der durch das umlaufende flüssige Medium im Wirbelzustand gehalten wird. Der Fermenter (25) wird bei einer Temperatur von 16°C und einem Druck von ca. 1,8 bar gefahren. Die in den Fermenter (25) eintretende gekühlte Würze wird in der Leitung (24) mit Luft gemischt, wobei ein Sauerstoffgehalt von 1,5 mg O₂/l Würze eingestellt wird. Die Luft wird über die Leitung (26) zugeführt. Das flüssige Medium hat im Fermenter (25) eine Aufenthaltszeit von ca. 5 Stunden, in der der größte Teil der vergärbaren Zucker in Alkohol umgewandelt wird. Als Biokatalysator hat sich in vorteilhafter Weise der in der DE-PS 3 704 478 beschriebene Katalysator wegen seiner guten mechanischen Festigkeit und seines ausgezeichneten Verhaltens in der Wirbelschicht bewährt.

Das flüssige Medium wird dem Fermenter (25) kontinuierlich entnommen und über die Leitung (41) in den Separator (42) geführt, wo die Abtrennung der im Fermenter (25) gebildeten freien Hefezellen erfolgt. Die abzentrifugierte Hefe wird über die Leitung (43) ausgetragen, während das feststofffreie flüssige Medium über die Leitung (44) in den Wärmeaustauscher (45) gelangt, wo es auf eine Temperatur von 62°C erhitzt wird. Im Wärmeaustauscher (45) hat das Medium eine Verweilzeit von ca. 20 Minuten. Ein Teilstrom des Ablaufs des Wärmeaustauschers (45) wird über die Leitung (46) dem Wärmeaustauscher (47) zugeführt und dort auf die Vergärungstemperatur abgekühlt sowie über die Leitung (48) in den Fermenter (25) zurückgeführt.

Der zweite Teilstrom des Ablaufs des Wärmeaustauschers (45) - also das heiße Bier - wird in zwei Teile geteilt. Der erste Teil gelangt über die Leitung (49) in die Strippkolonne (50), die bei einer mittleren Betriebstemperatur von 52°C betrieben wird. Die Strippkolonne (50) wird über die Leitung (51) mit Luft beschickt, die eine Temperatur von 70°C hat, die einen Wassergehalt aufweist, der 92 % des Sättigungs-Wassergehalts, bezogen auf die Luftaustrittstemperatur, beträgt, und die der Strippkolonne (50) in einer Menge von 4,5 Nm³/l Bier zugeführt wird. Der Wassergehalt der Luft wird in der Weise eingestellt, daß über die Leitung (52) eine Zufuhr von Wasserdampf in die Leitung (51) erfolgt. Das Bier hat in der Strippkolonne (50) eine Aufenthaltszeit von 12 Minuten. Der Luftstrom wird aus der Strippkolonne (50) über die Leitung (53) abgeführt. Aus dem Luftstrom können die kondensierbaren Bestandteile, insbesondere Äthanol und Wasser, in einem Kondensator abgetrennt werden; der Kondensator ist in der Zeichnung nicht dargestellt.

Das aus der Strippkolonne (50) über die Leitung (54) abfließende entalkoholisierte Produkt wird auf Atmosphärendruck (1 bar) entspannt und im Wärmeaustauscher (55) auf eine Temperatur von 2°C abgekühlt. Dem Wärmeaustauscher (55) wird über die Leitung (56) auch der zweite Teil des heißen Biers zugeführt, der den Wärmeaustauscher (45) verläßt. Auch dieser Teil wird im Wärmeaustauscher (55) auf eine Temperatur von 2°C abgekühlt. Das Mengenverhältnis der beiden Teile, welche den Wärmeaustauscher (45) über die Leitungen (49) und (56) verlassen, liegt so, daß ein Alkoholgehalt < 0,5 Vol.% resultiert.

Der Ablauf des Wärmeaustauschers (55) gelangt über die Leitung (57) in den Fermenter (58), der nach dem Prinzip des Schlaufenreaktors arbeitet, der den Biokatalysator enthält, der auch im Fermenter (25) zum Einsatz kommt und der bei einer Temperatur von 2°C sowie einem Druck von ca. 1,4 bar arbeitet. Im Fermenter (58) hat das alkoholarme Produkt eine Aufenthaltszeit von 1 Stunde, in der zwar eine geschmackliche Verbesserung, aber keine Erhöhung des Alkoholgehalts des Produkts eintritt. Der Ablauf des Fermenters (58) wird über die Leitung (59) einem Filter (60) zugeführt, das ggf. mit einem Filterhilfsmittel betrieben wird und in dem Trubstoffe sowie eventuell vorhandene freie Hefezellen aus dem alkoholarmen Bier abgetrennt werden. Das alkoholarme Bier hat einen Alkoholgehalt < 0,5 Vol.% und verläßt das Filter (60) über die Leitung (61), in der es mit CO₂ versetzt werden kann.

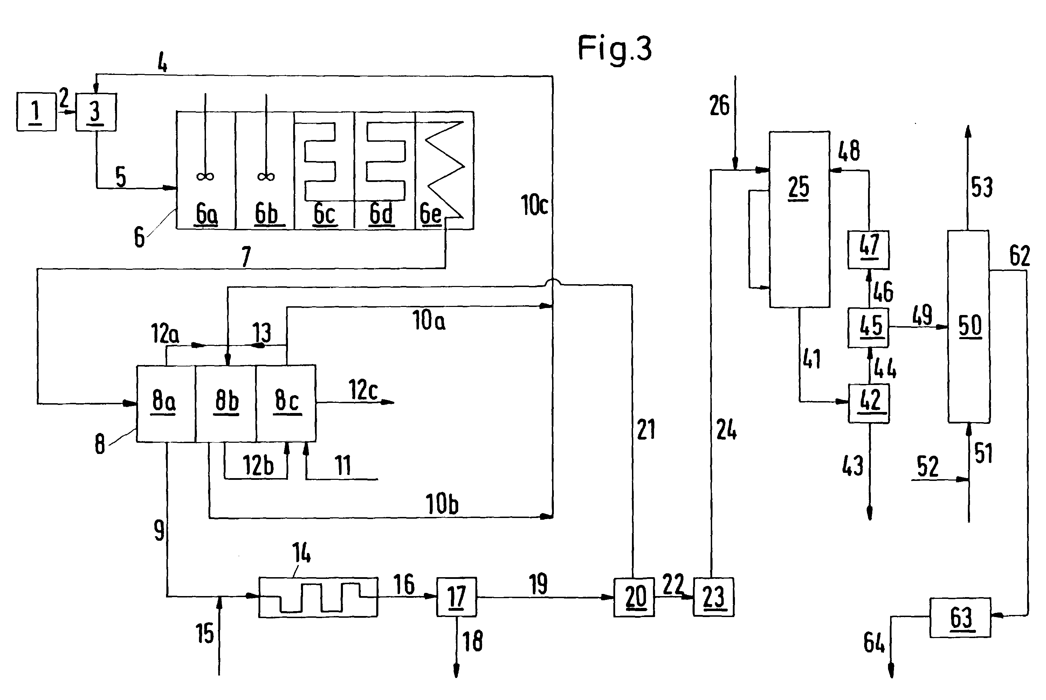

Das alkoholfreie Bier wird gemäß dem in Fig. 3 dargestellten Verfahren wie folgt hergestellt:

Die feststofffreie Würze wird gemäß dem in Fig. 1 dargestellten und vorstehend beschriebenen Teilprozeß hergestellt und im Wärmeaustauscher (23) auf die Vergärungstemperatur von 16°C abgekühlt. Aus dem Wärmeaustauscher (23) fließt die abgekühlte Würze über die Leitung (24) gemäß dem in Fig. 2 dargestellten und vorstehend beschriebenen Verfahren kontinuierlich in den Fermenter (25), wo der größte Teil der vergärbaren Zucker in Alkohol umgewandelt wird. Das flüssige Medium wird dem Fermenter (25) gemäß dem in Fig. 2 dargestellten und vorstehend beschriebenen Verfahren kontinuierlich entnommen und in den Fermenter (25) zurückgeführt.

Gemäß dem in Fig. 3 dargestellten Verfahren gelangt der zweite Teilstrom des Ablaufs des Wärmeaustauschers (45) - also das heiße Bier - ungeteilt über die Leitung (49) in die Strippkolonne (50), die bei einer mittleren Betriebstemperatur von 52°C betrieben wird. Die Strippkolonne (50) wird über die Leitung (51) mit Luft beschickt, die eine Temperatur von 70°C hat, die einen Wassergehalt aufweist, der 92 % des Sättigungs-Wassergehalts, bezogen auf die Luftaustrittstemperatur, beträgt, und die der Strippkolonne (50) in einer Menge von 4,5 Nm³/l Bier zugeführt wird. Der Wassergehalt der Luft wird in der Weise eingestellt, daß über die Leitung (52) eine Zufuhr von Wasserdampf in die Leitung (51) erfolgt. Das Bier hat in der Strippkolonne (50) eine Aufenthaltszeit von 12 Minuten. Der Luftstrom wird aus der Strippkolonne (50) über die Leitung (53) abgeführt. Aus dem Luftstrom können die kondensierbaren Bestandteile, insbesondere Alkohol und Wasser, in einem Kondensator abgetrennt werden; der Kondensator ist in der Zeichnung nicht dargestellt.

Das aus der Strippkolonne (50) über die Leitung (62) abfließende entalkoholisierte Produkt wird mit CO₂ gemischt, anschließend entspannt und im Wärmeaustauscher (63) auf eine Temperatur von 3°C abgekühlt. Das abgekühlte Produkt fließt über die Leitung (64) ab und wird in üblicher Weise weiterbehandelt. Das alkoholfreie Bier hat einen Alkoholgehalt < 0,05 Vol.%.

## Patentansprüche

1. Verfahren zur kontinuierlichen Herstellung von Bier, bei dem
a) zerkleinerte, stärkehaltige und ggf. gemälzte Rohstoffe mit Wasser eingemaischt und die Maische mindestens einem Reaktor kontinuierlich zugeführt wird, wobei die Temperatur der Maische vor Eintritt in mindestens einen der Reaktoren durch indirekten Wärmeaustausch stufenweise auf eine Endtemperatur von 75 bis 85°C angehoben wird, wobei die Verweilzeit in den Reaktoren 30 bis 90 Minuten beträgt und wobei die Maische in den einzelnen Reaktoren auf einem definierten Temperaturniveau gehalten wird,
b) der Treber aus der Maische in einem Dekanter kontinuierlich abgetrennt und anschließend mit dem Brauwasser in einem zweistufigen Dekanter ausgelaugt wird,
c) die feststofffreie, heiße Würze mit Hopfen oder Hopfenextrakt gemischt sowie kontinuierlich einem Durchflußreaktor zugeführt und auf eine Temperatur von 105 bis 140°C erhitzt wird sowie während der Durchgangs zeit durch den Reaktor von 2 bis 60 Minuten auf dieser Temperatur und auf einem Druck von 1,2 bis 3,6 bar gehalten wird,
d) die unter Druck stehende Würze einer Entspannungsverdampfung unterworfen, in einem Separator kontinuierlich von den Trubstoffen befreit und anschließend in einem Wärmeaustauscher auf die Vergärungstemperatur abgekühlt wird,
e) die abgekühlte Würze mit einem Sauerstoffgehalt von 0,5 bis 3,0 mg O₂/l mindestens einem als Schlaufenreaktor gestalteten Fermenter kontinuierlich zugeführt wird, der bei einer Temperatur von 6 bis 25°C sowie einem Druck von 1,5 bis 2 bar arbeitet, in dem die Würze eine mittlere Verweilzeit von 1 bis 40 Stunden hat sowie ständig im Kreislauf geführt wird und in dem sich ein Biokatalysator befindet, der eine biologisch aktive Hefe enthält,
f) während der Gärung kontinuierlich flüssiges Medium aus dem Fermenter abgezogen, zur Entfernung der darin befindlichen freien Hefezellen zentrifugiert, das enthefte flüssige Medium während 0,5 bis 30 Minuten auf 50 bis 90°C erhitzt und in zwei Teilströme heißen Bieres aufgeteilt wird,
g) ein Teilstrom des in der Verfahrensstufe f) anfallenden heißen Bieres abgekühlt und in den Fermenter zurückgeführt wird,
h) der zweite Teilstrom entweder teilweise oder ganz entalkoholisiert und als alkoholarmes oder alkoholfreies Bier abgegeben wird oder abgekühlt, filtriert, mit CO₂ versetzt und als alkoholhaltiges Bier abgegeben wird.

2. Verfahren nach Anspruch 1, dadurch gekennzeichnet, daß das Einmaischen der zerkleinerten Rohstoffe gemäß Verfahrensstufe a) in einer Kolloidmühle erfolgt.

3. Verfahren nach den Ansprüchen 1 bis 2, dadurch gekennzeichnet, daß die Verfahrensstufe c) bei einer Temperatur von 110 bis 125°C sowie einem Druck von 1,4 bis 2,3 bar durchgeführt wird.

4. Verfahren nach den Ansprüchen 1 bis 3, dadurch gekennzeichnet, daß die Entspannungsverdampfung gemäß Verfahrensstufe d) in zwei Stufen erfolgt, wobei in der ersten Stufe auf 1 bar und in der zweiten Stufe auf 0,3 bis 0,7 bar entspannt wird.

5. Verfahren nach den Ansprüchen 1 bis 4, dadurch gekennzeichnet, daß während der Entspannungsverdampfung gemäß Verfahrensstufe d) CO₂ zugeführt wird.

6. Verfahren nach den Ansprüchen 1 bis 5, dadurch gekennzeichnet, daß die Würze nach Abtrennung der Trubstoffe gemäß Verfahrensstufe d) in einen Lagerbehälter geführt wird und daß die dem Lagerbehälter entnommene Würze vor ihrem Eintritt in den Wärmeaustauscher der Stufe d) kurzfristig auf 60 bis 100°C erhitzt wird.

7. Verfahren nach den Ansprüchen 1 bis 6, dadurch gekennzeichnet, daß die Würze in der Verfahrensstufe e) nacheinander durch drei Fermenter geführt wird, wobei die Verweilzeit in den Fermentern insgesamt 10 bis 40 Stunden beträgt.

8. Verfahren nach den Ansprüchen 1 bis 6, dadurch gekennzeichnet, daß die Würze in der Verfahrensstufe e) durch einen Fermenter geführt wird, in dem die Verweilzeit 1 bis 8 Stunden beträgt.

9. Verfahren nach den Ansprüchen 1 bis 8, dadurch gekennzeichnet, daß der in der Verfahrensstufe e) verwendete Biokatalysator einen TiO₂-Gehalt von 5 bis 30 Gew.% aufweist sowie eine biologisch aktive Hefe und eine gelartige Matrix enthält, wobei die TiO₂-Teilchen einen Durchmesser von 0,1 bis 1 µm haben und der Katalysator kugelförmig ist.

10. Verfahren nach den Ansprüchen 1 bis 9, dadurch gekennzeichnet, daß das enthefte flüssige Medium gemäß Verfahrensstufe f) während 15 bis 20 Minuten auf 60 bis 65°C erhitzt wird.

11. Verfahren nach den Ansprüchen 1 bis 10, dadurch gekennzeichnet, daß der in der Verfahrensstufe f) anfallende zweite Teilstrom des heißen Bieres nochmal in zwei Teile geteilt wird, wobei aus dem ersten Teil des heißen Bieres der Alkohol kontinuierlich durch Strippen mit Luft und/oder Wasserdampf oder durch Entspannungsverdampfung entfernt und das entalkoholisierte Bier mit dem zweiten Teil verschnitten wird, wobei das Mengenverhältnis des ersten und zweiten Teils so gewählt wird, daß in der Mischung ein Alkoholgehalt < 2,5 Vol.%, vorzugsweise < 1 Vol.%, resultiert und daß das heiße alkoholarme Bier abgekühlt, filtriert und mit CO₂ versetzt wird.

12. Verfahren nach den Ansprüchen 1 bis 10, dadurch gekennzeichnet, daß aus dem in der Verfahrensstufe f) anfallenden zweiten Teilstrom des heißen Bieres der Alkohol kontinuierlich durch Strippen mit Luft und/oder Wasserdampf oder durch Entspannungsverdampfung entfernt wird und daß das alkoholfreie Bier abgekühlt, filtriert und mit CO₂ versetzt wird.

13. Verfahren nach den Ansprüchen 11 und 12, dadurch gekennzeichnet, daß der Alkohol aus dem 50 bis 60°C heißen Bier durch Strippen mit Luft in einer Kolonne entfernt wird, wobei die Luft mit Wasserdampf angereichert ist und eine Temperatur von 60 bis 100°C hat, und daß das alkoholfreie Bier anschließend auf 1 bar entspannt wird.

14. Verfahren nach den Ansprüchen 11 bis 13, dadurch gekennzeichnet, daß das alkoholarme oder alkoholfreie abgekühlte Bier einem als Schlaufenreaktor gestalteten Fermenter zugeführt wird, der bei einer Temperatur von 0 bis 6°C sowie einem Druck von 1,2 bis 1,5 bar arbeitet, in dem das alkoholfreie Bier eine Aufenthaltszeit von 0,2 bis 2 Stunden hat und in dem sich ein Biokatalysator befindet, der mit dem in der Verfahrensstufe e) verwendeten Biokatalysator identisch ist.

## Claims

1. A process for the continuous production of beer, in which
a) comminuted, starch-containing and optionally malted raw materials are mashed with water and the mash is supplied continuously to at least one reactor, the temperature of the mash before entry into at least one of the reactors being raised in steps to a final temperature of 75 to 85°C by indirect heat exchange, the dwell time in the reactors being 30 to 90 minutes and the mash in the individual reactors being kept at a defined temperature level,
b) the spent grains are continuously separated off from the mash in a decanter and then are leached out with the brewing water in a two-stage decanter,
c) the solids-free, hot wort is mixed with hops or hop extract and continuously supplied to a continuous-flow reactor and heated to a temperature of 105 to 140°C and during the passage time through the reactor of 2 to 60 minutes is kept at this temperature and at a pressure of 1.2 to 3.6 bar,
d) the wort, which is under pressure, is subjected to flash evaporation, is freed continuously of the turbidity-causing solids in a separator and then is cooled in a heat exchanger to the fermenting temperature,
e) the cooled wort with an oxygen content of 0.5 to 3.0 mg O₂/l is supplied continuously to at least one fermenter designed as a loop reactor, which fermenter operates at a temperature of 6 to 25°C and a pressure of 1.5 to 2 bar, in which the wort has an average dwell time of 1 to 40 hours and is continuously circulated and in which a biocatalyst is located which contains a biologically active yeast,
f) during fermentation liquid medium is continuously withdrawn from the fermenter, is centrifuged in order to remove the free yeast cells located therein, the de-yeasted liquid medium is heated for 0.5 to 30 minutes to 50 to 90°C and is divided into two partial streams of hot beer,
g) one partial stream of the hot beer produced in process stage f) is cooled and returned to the fermenter,
h) the second partial stream is either partly or completely dealcoholised and is given off as low-alcohol or alcohol-free beer, or is cooled, filtered, has CO₂ added thereto and is given off as alcohol-containing beer.

2. A process according to Claim 1, characterised in that the mashing of the comminuted raw materials according to process stage a) is effected in a colloid mill.

3. A process according to Claims 1 to 2,
characterised in that process stage c) is effected at a temperature of 110 to 125°C and a pressure of 1.4 to 2.3 bar.

4. A process according to Claims 1 to 3,
characterised in that the flash evaporation according to process stage d) is effected in two stages, with pressure relief being effected to 1 bar in the first stage and to 0.3 to 0.7 bar in the second stage.

5. A process according to Claims 1 to 4,
characterised in that CO₂ is supplied during the flash evaporation according to process stage d).

6. A process according to Claims 1 to 5,
characterised in that the wort after separation of the turbidity-causing solids according to process stage d) is passed into a storage vessel and that the wort removed from the storage vessel is briefly heated to 60 to 100°C before entering the heat exchanger of stage d).

7. A process according to Claims 1 to 6,
characterised in that the wort in process stage e) is passed in succession through three fermenters, the dwell time in the fermenters being a total of 10 to 40 hours.

8. A process according to Claims 1 to 6,
characterised in that the wort in process stage e) is passed through a fermenter in which the dwell time is 1 to 8 hours.

9. A process according to Claims 1 to 8,
characterised in that the biocatalyst used in process stage e) has a TiO₂ content of 5 to 30% by weight and contains a biologically active yeast and a gel-like matrix, the TiO₂ particles having a diameter of 0.1 to 1 µm and the catalyst being spherical.

10. A process according to Claims 1 to 9,
characterised in that the de-yeasted liquid medium according to process stage f) is heated to 60 to 65°C for 15 to 20 minutes.

11. A process according to Claims 1 to 10,
characterised in that the second partial stream of the hot beer produced in process stage f) is once again divided into two parts, with the alcohol being continuously removed from the first part of the hot beer by stripping with air and/or water vapour or by flash evaporation, and the dealcoholised beer being blended with the second part, the proportion of the first and second parts being selected such that an alcohol content of < 2.5% by volume, preferably < 1% by volume, results in the mixture and that the hot, low-alcohol beer is cooled, filtered and has CO₂ added to it.

12. A process according to Claims 1 to 10,
characterised in that the alcohol is continuously removed from the second partial stream of the hot beer produced in process stage f) by stripping with air and/or water vapour or by flash evaporation, and that the alcohol-free beer is cooled, filtered and has CO₂ added thereto.

13. A process according to Claims 11 and 12,
characterised in that the alcohol is removed from the beer, which is at a temperature of 50 to 60°C, by stripping with air in a column, the air being enriched with water vapour and being at a temperature of 60 to 100°C, and that the alcohol-free beer is then pressure-relieved to 1 bar.

14. A process according to Claims 11 to 13,
characterised in that the low-alcohol or alcohol-free cooled beer is supplied to a fermenter designed as a loop reactor, which fermenter operates at a temperature of 0 to 6°C and a pressure of 1.2 to 1.5 bar, in which the alcohol-free beer has a dwell time of 0.2 to 2 hours and in which a biocatalyst is located which is identical to the biocatalyst used in process stage e).

## Revendications

1. Procédé de production de bière en continu, qui consiste :
a) à tremper dans de l'eau des matières premières fragmentées, amylacées et éventuellement maltées et à envoyer la trempe en continu à au moins à un réacteur, la température de la trempe avant l'entrée dans le au moins un réacteur étant portée par paliers, par échange de chaleur indirecte, à une température finale de 75 à 85 °C, la durée de séjour dans les réacteurs étant de 30 à 90 mn et la trempe étant maintenue dans les réacteurs à un niveau de température défini,
b) à séparer en continu la drêche de la trempe dans un décanteur et ensuite à la lixivier par l'eau de brassage dans un décanteur à deux étages,
c) à mélanger le moût chaud et exempt de matière solide à du houblon ou à de l'extrait de houblon, ainsi qu'à l'envoyer en continu à un réacteur à passage direct et à le porter à une température de 105 à 140°C, tout en le maintenant, pendant la durée de passage dans le réacteur, de 2 à 60 minutes à cette température et sous une pression de 1,2 à 3,6 bar,
d) à soumettre le moût sous pression à une évaporation avec détente, à le débarrasser en continu dans un séparateur des dépôts et ensuite à le refroidir dans un échangeur de chaleur jusqu'à la température de fermentation,
e) à envoyer le moût refroidi et ayant une teneur en oxygène de 0,5 à 3,0 mg de 02/litre en continu au moins à un fermenteur conformé en réacteur à boucle et fonctionnant à une température de 6 à 25°C ainsi que sous une pression de 1,5 à 2 bar, dans lequel le moût a une durée de séjour moyenne de 1 à 40 heures, dans lequel il est mis constamment en recirculation en circuit fermé et dans lequel se trouve un catalyseur biologique qui renferme une levure active biologiquement,
f) pendant la fermentation, à soutirer en continu du milieu liquide du fermenteur, à le centrifuger pour en éliminer les cellules de levure libres qui s'y trouvent, à porter le milieu liquide dont on a enlevé la levure pendant 0,5 à 30 minutes à 50 à 80°C et à le répartir en deux courants partiels de bière chaude,
g) à refroidir un courant partiel de la bière chaude se produisant au stade f) du procédé et à le retourner au fermenteur,
h) à désalcooliser en tout ou partie le second courant partiel et à le soutirer en tant que bière pauvre en alcool ou sans alcool ou à le refroidir, à le filtrer, à le mélanger à du CO₂ et à le soutirer en tant que bière alcoolisée.

2. Procédé suivant la revendication 1, caractérisé en ce qu'il consiste à effectuer la trempe des matières premières fragmentées suivant le stade a) du procédé dans un concasseur à colloïdes.

3. Procédé suivant l'une des revendications 1 ou 2, caractérisé en ce qu'il consiste à effectuer le stade c) du procédé à une température de 110 à 125°C ainsi que sous une pression de 1,4 à 2,3 bar.

4. Procédé suivant l'une des revendications 1 à 3, caractérisé en ce qu'il consiste à effectuer l'évaporation avec détente suivant le stade d) du procédé dans deux étages en détendant dans le premier étage jusqu'à 1 bar et dans le second étage jusqu'à 0,3 à 0,7 bar.

5. Procédé suivant l'une des revendications 1 à 4, caractérisé en ce qu'il consiste à envoyer, pendant l'évaporation avec détente suivant le stade d) du procédé, du CO₂.

6. Procédé suivant l'une des revendications 1 à 5, caractérisé en ce qu'il consiste à envoyer le moût, après la séparation des matières de dépôt suivant le stade d) du procédé, dans une cuve de stockage et à porter le moût prélevé de la cuve de stockage brièvement entre 60 et 100°C avant son entrée dans l'échangeur de chaleur du stade d).

7. Procédé suivant l'une des revendications 1 à 7, caractérisé en ce qu'il consiste à envoyer le moût dans le stade e) du procédé successivement dans trois fermenteurs, la durée de séjour dans les fermenteurs étant au total de 10 à 40 heures.

8. Procédé suivant l'une des revendications 1 à 6, caractérisé en ce qu'il consiste à envoyer le moût au stade e) du procédé dans un fermenteur dans lequel la durée de séjour est de 1 à 8 heures.

9. Procédé suivant l'une des revendications 1 à 8, caractérisé en ce que le catalyseur biologique, utilisé au stade e) du procédé, à une teneur en TiO₂ de 5 à 30% en poids et renferme une levure active biologiquement et une matrice de type gel, les particules de TiO₂ ayant un diamètre de 0,1 à 1 µm et le catalyseur étant sous forme de billes.

10. Procédé suivant l'une des revendications 1 à 9, caractérisé en ce qu'il consiste à porter le milieu liquide, dont on a enlevé la levure suivant le stade f) du procédé, entre 60 et 65°C pendant 15 à 20 minutes.

11. Procédé suivant l'une des revendications 1 à 10, caractérisé en ce qu'il consiste à subdiviser le second courant partiel de la bière chaude se produisant au stade f) du procédé en deux parties, l'alcool étant éliminé de la première partie de la bière chaude en continu par entraînement à l'air/ou à la vapeur d'eau ou par évaporation avec détente et la bière désalcoolisée étant diluée avec la seconde partie, le rapport quantitatif de la première et de la seconde partie étant tel que l'on obtient dans le mélange une teneur en alcool inférieur à 2,5% en volume et de préférence inférieur à 1% en volume, et à refroidir la bière chaude pauvre en alcool, à la filtrer et à la mélanger à du CO₂.

12. Procédé suivant l'une des revendications 1 à 10, caractérisé en ce qu'il consiste à enlever, du second courant partiel de la bière chaude se produisant au stade f) du procédé, l'alcool en continu par entraînement à l'air/ou à la vapeur d'eau ou par évaporation avec détente et à refroidir la bière sans alcool, à la filtrer et à la mélanger à du CO₂.

13. Procédé suivant l'une des revendications 11 et 12, caractérisé en ce qu'il consiste à enlever l'alcool de la bière chaude ayant une température de 50 à 60°C par entraînement à l'air dans une colonne, l'air s'enrichissant en vapeur d'eau et ayant une température de 60 à 100°C et à détendre la bière sans alcool ensuite à 1 bar.

14. Procédé suivant l'une des revendications 1 à 13, caractérisé en ce qu'il consiste à envoyer la bière refroidie, pauvre en alcool ou sans alcool, à un fermenteur conformé en réacteur à boucle, qui fonctionne à une température de 0 à 6°C ainsi que sous une pression de 1,2 à 1,5 bar et dans lequel la bière sans alcool a une durée de séjour de 0,2 à 2 heures et dans lequel se trouve un catalyseur biologique qui est identique au catalyseur biologique utilisé au stade e) du procédé.
